# EUROPÄISCHE PATENTANMELDUNG

(11) **EP 3 075 329 A1**
(43) Veröffentlichungstag der Anmeldung: **05.10.2016**
(21) Anmeldenummer: 16159353.8
(22) Anmeldetag: 09.03.2016
(51) Int. Cl.: A61B 17/12, A61B 17/24

(54) **VORRICHTUNG ZUR STILLUNG VON BLUTUNGEN**

(30) Priorität: 26.03.2015 DE 202015101546 U
(71) Anmelder: Hemmel, Gunter, 94350 Falkenfels (DE)
(72) Erfinder: Hemmel, Gunter, 94350 Falkenfels (DE)
(74) Vertreter: advotec.

(57) **Zusammenfassung**

Die Erfindung betrifft eine Vorrichtung zum Stillen von Blutungen, insbesondere von Blutungen im Nasen- und/oder Rachenraum, mit einem beidseitig offenen flexiblen Atemschlauch (2) sowie einem den Atemschlauch (2) mindestens abschnittsweise umhüllenden, mindestens teilweise ausdehnbaren Hüllelement (3, 21), wobei zur Ausdehnung des Hüllelements Medium, insbesondere Luft in einen Raum (14, 29) zwischen dem flexiblen Atemschlauch (2) und dem Hüllelement (21) oder - im Falle eines zweilagig ausgebildeten Hüllelements (3) - zwischen einer äußeren Lage (10) und einer inneren Lage (9) des Hüllelements (3) abwechselnd einbringbar und wieder ausbringbar ist.

## Beschreibung

Die vorliegende Erfindung betrifft eine Vorrichtung zum Stillen von Blutungen, insbesondere von Blutungen im Nasen- und/oder Rachenraum.

Blutungen in der Nasenhöhle oder dem Nasen-Rachenraum treten häufig nach operativen Eingriffen, beispielsweise nach dem Entfernen der Polypen auf. Diese Blutungen sind teilweise heftig und müssen daher umgehend gestillt werden, um einen zu hohen Blutverlust zu vermeiden.

Zum Stillen derartiger Blutungen wird in der Regel so vorgegangen, dass Verbandsmull, Tampons aus Zellstoff oder ähnliches Material in die Nase eingeführt wird. Dieses Material wird so tief in den Nasenraum geschoben, bis es die blutende Stelle erreicht hat und auf diese Art und Weise die Blutung stillen kann.

Ein derartiges Vorgehen beim Stillen von Blutungen im Nasen-Rachenraum, bei dem die Nase mit Verbandsmull, Tampons etc. vollgestopft wird, ist jedoch mit diversen Nachteilen verbunden. So ist es für den Patienten sehr unangenehm, derartiges Material über einen längeren Zeitraum im Nasenraum zu tragen, da sich hier im Laufe der Zeit ein hoher Druck aufbaut. Zum anderen entstehen oftmals Probleme beim Entfernen derartiger Tamponagen, da Blutungen beim Herausziehen des Materials wieder provoziert werden können. Das anhaftende Material kann Gewebe mitreißen und so die betroffene Stelle wieder öffnen.

Ein weiterer gravierender Nachteil besteht darin, dass der Patient während der gesamten Zeit, in der sich das Material in der Nase befindet, nicht durch Nase atmen kann. Dies ist insbesondere in der Nacht äußerst störend und verhindert oftmals ein Schlafen des Patienten.

Um die geschilderten Nachteile wenigstens teilweise zu vermeiden, wurden in der Vergangenheit Vorrichtungen entwickelt, die die genannten Tamponagen ersetzen sollen. So beschreibt die Patentschrift DE 430 234 eine Vorrichtung zum Stillen von Blutungen im Nasenraum, welche im Wesentlichen einen aufblasbaren Ballon am Ende eines Rohres aufweist. Zum Stillen einer Blutung wird der aufblasbare Ballon im erschlafften Zustand in die Nase eingeführt und dann mittels einer Handpumpe aufgepumpt. Er erweitert sich sodann im Nasenraum und soll so Blutungen stillen. Zum Herausnehmen wird die Luft wieder abgelassen, sodass die Vorrichtung einfach wieder aus der Nase herausgezogen werden kann. Bei dieser Vorrichtung besteht jedoch nach wie vor das Problem, dass der Patient nach dem Einführen und Aufblasen des aufblasbaren Ballons nicht mehr durch die Nase atmen kann.

Eine ähnliche Vorrichtung wird auch in DE 2435183 beschrieben. Auch hier besteht das Problem, dass im Einsatzzustand der Vorrichtung ein Atmen durch die Nase nicht möglich ist.

Der vorliegenden Erfindung liegt die Aufgabe zugrunde, eine Vorrichtung zum Stillen von Blutungen zur Verfügung zu stellen, welche die Nachteile aus dem Stand der Technik überwindet.

Diese Aufgabe wird gelöst durch eine Vorrichtung, umfassend einen beidseitig offenen flexiblen Atemschlauch sowie ein den Atemschlauch mindestens abschnittsweise umhüllendes mindestens teilweise ausdehnbares Hüllelement, wobei zum Ausdehnen des Hüllelements Medium, insbesondere Luft in einen Raum zwischen dem flexiblen Atemschlauch und dem Hüllelement oder im Falle eines zweilagig ausgebildeten Hüllelements zwischen einer äußeren und einer inneren Lage des Hüllelements abwechselnd ein- und wieder ausbringbar ist.

Mit der erfindungsgemäßen Vorrichtung können sämtliche o. g. Nachteile überwunden werden. So ist es zum einen möglich, die erfindungsgemäße Vorrichtung in einem noch nicht aufgeblasenen Zustand in die Nase einfach einzuführen. Befindet sich die Vorrichtung dann in der geeigneten Position, kann durch Einbringen von Medium in einen der genannten Räume das Hüllelement ausgeweitet werden, sodass es im Inneren des Nasen- oder Rachenraums zu einer Ausdehnung des Hüllelements und dadurch zu einer Volumenvergrößerung der Vorrichtung kommt. Auf diese Art und Weise kann dann auf die blutende Stelle gedrückt und Blutungen gestillt werden. Ein gravierender Vorteil der erfindungsgemäßen Vorrichtung besteht darin, dass ein Patient auch nach dem Einbringen von Medium, also nach dem Ausdehnen des Hüllelements im Nasenraum weiter durch die Nase atmen kann. Dies wird durch den beidseitig offenen flexiblen Atemschlauch ermöglicht, durch den zu jedem Zeitpunkt durch die Nase ein- und ausgeatmet werden kann.
Zum Herausnehmen der Vorrichtung aus der Nase des Patienten wird vorher das eingebrachte Medium wieder ausgelassen. Dabei kehrt das Hüllelement in seinen ursprünglichen, erschlafften Zustand zurück, wodurch eine Verringerung des Volumens der Vorrichtung erreicht wird. Die Vorrichtung kann dann wieder einfach aus der Nase entnommen werden, ohne Verletzungen im Körperinneren zu verursachen.

Mit Vorteil ist der Atemschlauch im Wesentlichen aus Silikon gefertigt. Dieses Material ist besonders flexibel und dadurch geeignet, in den Nasen- oder Rachenraum eines Patienten eingeführt zu werden. Der flexible Atemschlauch kann eine Lage aus Gewebe im Schlauchinneren enthalten.

Mit Vorteil ist das Hüllelement im Wesentlichen aus Latex gefertigt. Dieses Material ist besonders dehnfähig und zudem robust und reißfest.

Vorzugsweise ist das Hüllelement mit dem Atemschlauch mindestens abschnittsweise, insbesondere an seinen Endbereichen fest verbunden, insbesondere verklebt oder mittels eines Halterings (z. B. Schelle) verbunden. Hierdurch wird erreicht, dass sich das Hüllelement beim Einbringen von Medium, insbesondere Luft in den Raum zwischen dem Hüllelement und dem Atemschlauch bzw. einer äußeren und einer inneren Lage des Hüllelements nur in seiner Quererstreckung, und kaum in seiner Längserstreckung ausdehnt. Hierdurch wird insbesondere erreicht, dass die Enden des Atemschlauches offen bleiben und nicht durch das Hüllelement ganz oder teilweise bedeckt werden.

Bei einer besonders bevorzugten Ausführungsform der erfindungsgemäßen Vorrichtung ist dieser ein Schlauch zum Einbringen von Medium, insbesondere Luft zugeordnet, welcher vorzugsweise mit einer Pumpe zum Einpumpen des Mediums verbunden ist. Diese Pumpe kann vorteilhafterweise eine einfache Handpumpe sein, mit welcher Medium in einen der o. g. Räume einpumpbar ist.

Mit Vorteil weist die erfindungsgemäße Vorrichtung ein Ventil, insbesondere ein Rücklaufventil auf. Ein derartiges Ventil kann beispielsweise in einer Abschlusskalotte oder dem o. g. Schlauch zum Einbringen von Medium angeordnet sein. Mit Hilfe eines derartigen Ventils kann auf einfache Art und Weise Medium, insbesondere Luft in den Raum zwischen dem flexiblen Atemschlauch und dem Hüllelement oder zwischen einer äußeren und einer inneren Lage des Hüllelements ein- und wieder ausgebracht werden.

Bei einer Weiterbildung der erfindungsgemäßen Vorrichtung ist im Bereich eines distalen, im Einsatzzustand außerhalb des Körpers, insbesondere der Nase befindlichen Endes des Hüllelements ein Abschlussring angeordnet, welcher den Atemschlauch umgibt und mit diesem bzw. einer inneren Lage des Hüllelements sowie mit einer äußeren Lage des Hüllelements fest verbunden, insbesondere verklebt oder mittels eines Halterings (z. B. Schelle) verbunden ist, wobei der Abschlussring eine Öffnung zum Einbringen von Medium aufweist. Ein derartiger Abschlussring stellt eine gute Verbindungsstelle zwischen dem Hüllelement und einem Schlauch zum Einbringen von Medium dar.

Mit Vorteil ist der Abschlussring doppelwandig ausgebildet, wobei die Wände an ihrem einen Ende frei und an ihrem anderen Ende über eine ringförmige Abschlussplatte miteinander verbunden sind, welche Abschlussplatte vorzugsweise eine Aussparung zum Ein- und Ausbringen von Medium aufweist. Mit einem derartig ausgebildeten Abschlussring ist eine besonders vorteilhafte Anbindung der erfindungsgemäßen Vorrichtung an einen Schlauch und eine mit dem Schlauch verbundene Pumpe möglich.

Weitere Merkmale der Erfindung ergeben sich aus der nachfolgenden Beschreibung von bevorzugten Ausführungsbeispielen der Erfindung in Verbindung mit den Zeichnungen und den Unteransprüchen. Hierbei können die einzelnen Merkmale für sich allein oder in Kombination miteinander verwirklicht sein.

In den Zeichnungen zeigen:
- Fig. 1:: Eine perspektivische Darstellung einer erfindungsgemäßen Vorrichtung im nicht aufgeblasenen Zustand (Ruhezustand);
- Fig. 2:: eine weitere Darstellung der Vorrichtung von Fig. 1;
- Fig. 3:: einen Längsschnitt durch die Vorrichtung von Fig. 1;
- Fig. 4:: eine weitere Längsschnittdarstellung der Vorrichtung von Fig. 1;
- Fig. 5:: eine perspektivische Darstellung einer weiteren Ausführungsform der erfindungsgemäßen Vorrichtung im aufgeblasenen Zustand (Einsatzzustand);
- Fig. 6:: einen Längsschnitt durch die Vorrichtung von Fig. 5;

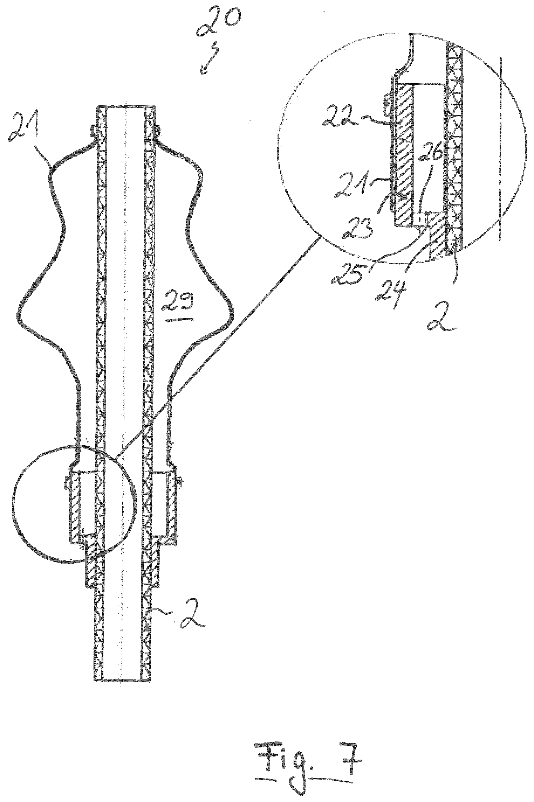

- Fig. 7:: eine weitere Längsschnittdarstellung der Vorrichtung von Fig. 5.

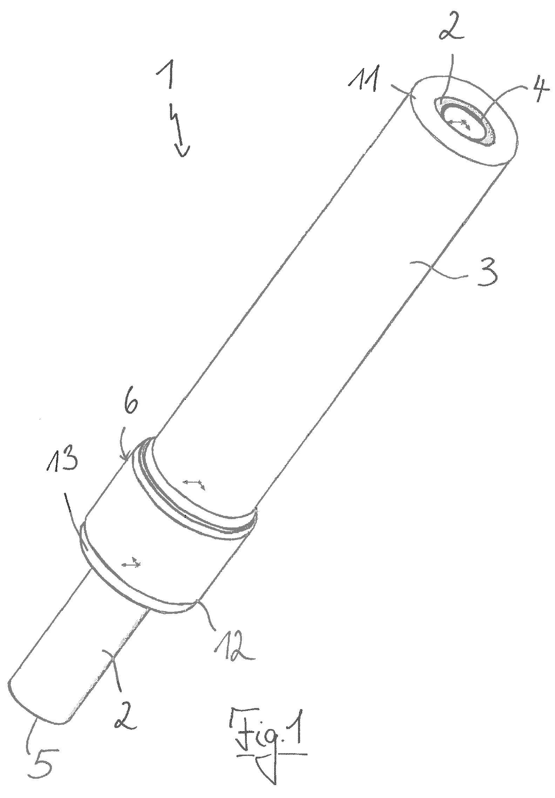

Fig. 1 zeigt eine erfindungsgemäße Vorrichtung 1 zum Stillen von Blutungen im Nasenraum. Die Vorrichtung 1 umfasst einen beidseitig offenen flexiblen Atemschlauch 2 aus Silikon sowie einen den Atemschlauch 2 teilweise umhüllenden Mantel 3 aus Latex. Der Latexmantel 3 erstreckt sich von einem Ende 4 des Atemschlauches 2, welches Ende zum Stillen von Blutungen im Nasenraum in die Nase eingeschoben wird, in Richtung des distalen Endes 5 und umhüllt in etwa dreiviertel des Atemschlauches 2. Des Weiteren umfasst die Vorrichtung 1 einen Abschlussring 6, welcher vom Latexmantel 3 nahezu völlig umhüllt ist.

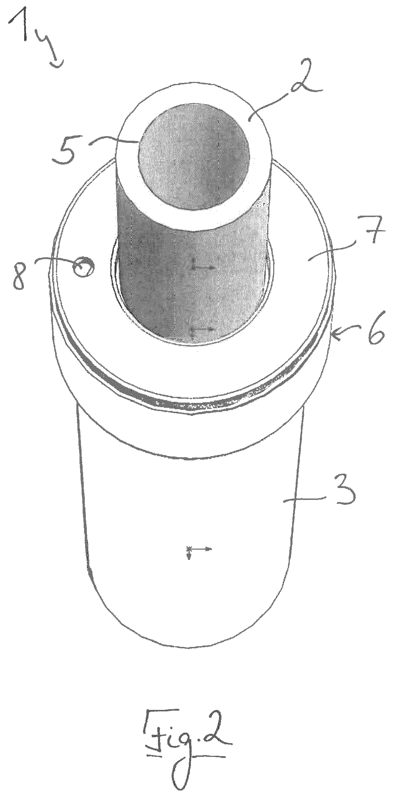

Fig. 2 zeigt eine weitere Darstellung der Vorrichtung 1. In dieser Darstellung ist gut zu erkennen, dass der Abschlussring 6 eine ringförmige Abschlussscheibe 7 aufweist, sodass der Abschlussring 6 nach außen hin abgeschlossen ist. In der Abschlussscheibe 7 ist lediglich eine kreisrunde Aussparung eingebracht, welche zum Anschließen eines Schlauches zum Einbringen von Luft in die Vorrichtung 1 dient. Der Abschlussring 6 sowie die Abschlussscheibe 7 sind in dieser Ausführungsform aus thermoplastischem Kunststoff gefertigt.

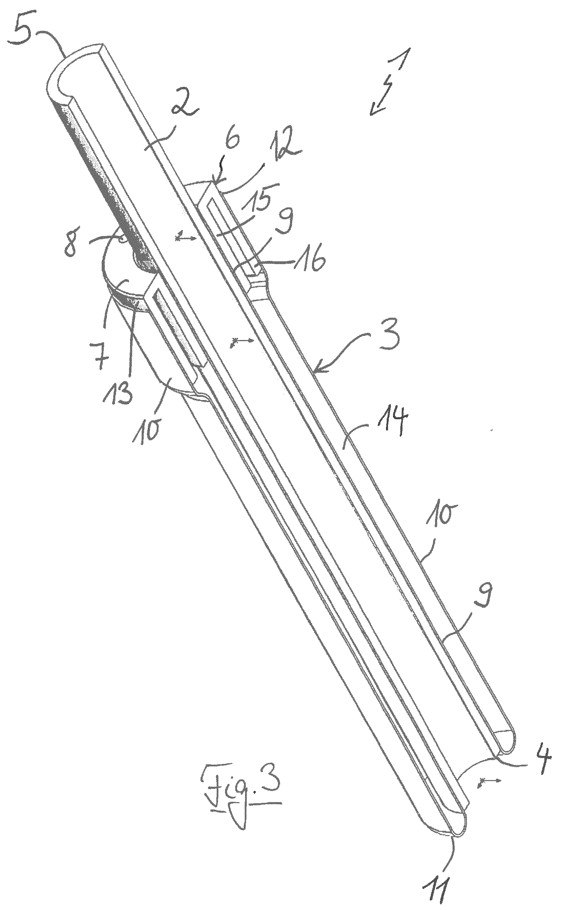

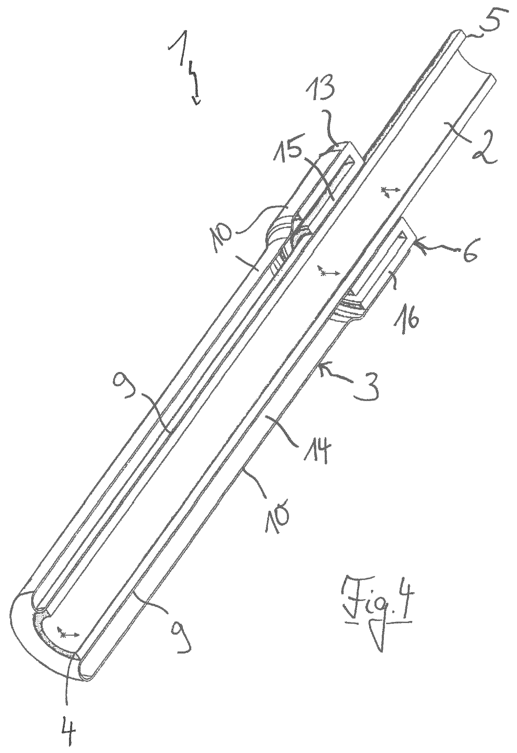

Fig. 3 und 4 zeigen einen Längsschnitt durch die Vorrichtung 1. In diesen Figuren ist gut zu erkennen, dass der Latexmantel 3 in dieser Ausführungsform zweilagig ausgeführt ist. So umfasst der Mantel 3 eine innere Lage 9 sowie eine äußere Lage 10, die an einem Ende 11 des Mantels 3 miteinander verbunden sind. An ihrem distalen Ende 12 sind die Lagen 9 und 10 nicht miteinander verbunden. Im Bereich des distalen Endes 12 umschließen die beiden Lagen 9 und 10 den Abschlussring 6 nahezu vollständig. Lediglich ein kleiner Abschnitt 13 des Abschlussringes 6 liegt frei. Die innere Lage 9 des Latexmantels 3 ist mit dem Atemschlauch 2 verklebt. Auch der Abschlussring 6 ist mit der inneren Lage 9 sowie der äußeren Lage 10 des Latexmantels 3 mittels Silikon verklebt.
Zwischen der inneren Lage 9 sowie der äußeren Lage 10 des Mantels 3 befindet sich also ein geschlossener Raum 14, in welchen Luft einpumpbar ist.

Der Abschlussring 6 ist doppelwandig ausgebildet, und weist eine innere Wand 15 sowie eine äußere Wand 16 auf. Die Wände 15 und 16 sind an einem Ende frei und an ihrem anderen Ende über die ringförmige Abschlussscheibe 7 miteinander verbunden. Durch diese Ausbildung des Abschlussringes 6 kann über die Öffnung 8 in der Abschlussscheibe 7 mittels einer hier nicht dargestellten Pumpe, die mit einem ebenfalls hier nicht dargestellten Schlauch mit der Öffnung 8 in der Abschlussscheibe 7 verbunden ist, eingebracht werden. Die eingepumpte Luft durchströmt zunächst den Raum zwischen der inneren Wand 15 und der äußeren Wand 16 des Abschlussringes 6 und strömt dann weiter in den Raum zwischen der inneren Lage 9 sowie der äußeren Lage 10 des Latexmantels 3. Das Einpumpen der Luft erfolgt dann, wenn die Vorrichtung 1 die gewünschte Lage im Nasenraum eines Patienten gefunden hat. Zum Einschieben der Vorrichtung 1 in die Nase eines Patienten ist der Latexmantel 3 noch nicht aufgepumpt, sodass die Vorrichtung 1 einen möglichst geringen Durchmesser aufweist. Zur erleichterten Einführung kann die Vorrichtung mit Gleitmittel versehen werden. Nach dem Positionieren der Vorrichtung 1 im Nasenraum eines Patienten wird Luft über die Öffnung 8 in der Abschlussscheibe 7 eingepumpt. Dabei dehnt sich die äußere Lage 10 des Latexmantels 3 an den Stellen besonders aus, an denen sich freie Räume im Naseninneren befinden. Durch die Ausdehnung des Mantels 3 wird dieser (die äußere Lage 10) an die blutende Stelle im Nasenraum gepresst, wodurch die Blutung schnell gestillt werden kann.
Der hier nicht dargestellte Schlauch, durch den Luft in die Vorrichtung 1 geleitet wird, weist in der Regel ein Rückschlagventil auf, sodass die eingepumpte Luft über eine gewünschte Zeit im Raum 14 zwischen den Lagen 9 und 10 verbleibt. Ist die Blutung gestillt, kann die Luft im Raum 14 wieder entlassen werden. Dies geschieht durch einen aktiven Vorgang, beispielsweise durch Betätigen eines Kugelhahns, Ventils oder dergleichen. Ist die Luft schließlich entwichen, kann die Vorrichtung 1, die jetzt wieder den Ausgangszustand erreicht hat, einfach aus dem Nasenraum entnommen werden.

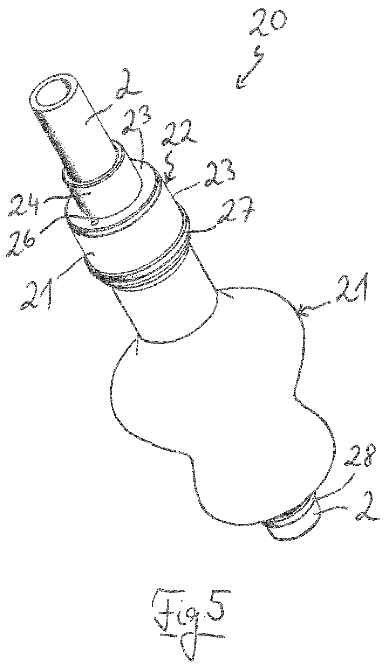

Fig. 5 zeigt eine weitere Ausführungsform einer erfindungsgemäßen Vorrichtung 20 in einem Einsatzzustand in einem Nasenraum. Der Übersicht halber wurde die Situation im Nasenraum nicht dargestellt. Auch die Vorrichtung 20 umfasst einen Atemschlauch 2 aus Silikon. Ferner umfasst die Vorrichtung 1 einen Latexmantel 21 sowie einen Abschlussring 22. Der Abschlussring 22 weist einen Basiskörper 23 sowie einen konzentrisch am Basiskörper 23 angeformten Leitungsabschnitt 24 auf. Der Basisabschnitt 23 ist durch eine Abschlussscheibe 25, in welche eine Öffnung 26 eingebracht ist, abgeschlossen. Der Latexmantel 21 ist im Bereich des Abschlussringes 22 mit diesem über einen Haltering 27 verbunden. Im Bereich des anderen Endes ist der Latexmantel 21 ebenfalls über einen Haltering 28 fest mit dem Atemschlauch 2 verbunden.

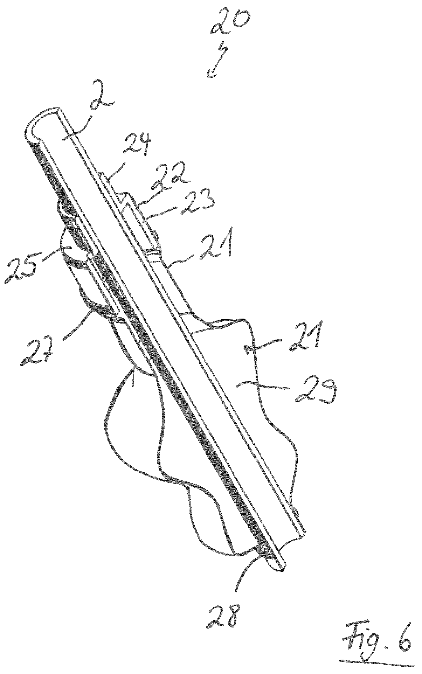

In Fig. 6 ist gut zu erkennen, dass der Latexmantel 21 der Vorrichtung 20 einlagig ausgebildet ist. Der Latexmantel 21 ist lediglich über die Halteringe 27 und 28 mit dem Atemschlauch 2 bzw. dem Abschlussring 22 verbunden. Ansonsten besteht keine feste Verbindung zwischen dem Latexmantel 21 und dem Atemschlauch 2. Dadurch besteht ein Raum 29 zwischen dem Latexmantel 21 und dem Atemschlauch 2, in den Luft eingepumpt werden kann.

Die Luft, die in dieser Ausführungsform ebenfalls über einen mit einer Pumpe verbundenen Schlauch, der in die Öffnung 26 der Abschlussscheibe 25 eingebracht wird, eingepumpt. Die eingepumpte Luft durchströmt zunächst den Raum zwischen dem Atemschlauch 2 und dem Basisabschnitt 23 des Abschlussringes 22. Von dort aus strömt die Luft weiter in den Raum zwischen dem Atemschlauch 2 und dem Latexmantel 21, wodurch sich der Latexmantel 21 ausdehnt und sich so an die Wände der Nasenhöhle anschmiegt. Hierdurch werden die Blutungen im Nasenraum gestillt. Vor dem Herausnehmen wird auch hier die Luft aus dem Raum zwischen dem Atemschlauch 2 und dem Latexmantel 21 entlassen, wodurch sich der Durchmesser der Vorrichtung 1 wieder minimiert.

Ein wichtiges Merkmal bei beiden Vorrichtungen 1 und 20 ist, dass der Atemschlauch 2 sowohl im Ruhezustand (nicht aufgeblasener Zustand) als auch im Einsatzzustand (aufgeblasener Zustand) an beiden Enden offen ist. Hierbei ist es wichtig zu vermeiden, dass der Latexmantel 3 bzw. 21 beim Einbringen von Luft die Schlauchöffnung verschließt. Durch den beidseitig offenen Atemschlauch kann ein Patient auch im Einsatzzustand der Vorrichtung bequem durch die Nase atmen.

Es versteht sich, dass der Atemschlauch auch aus anderen flexiblen Materialien außer Silikon gefertigt sein kann, die für derartige medizinische Eingriffe geeignet sind (z. B. Gummi).
Auch der Mantel 3 bzw. 21 kann aus anderen flexiblen, ausdehnbaren Materialien gefertigt sein.

## Patentansprüche

1. Vorrichtung zum Stillen von Blutungen, insbesondere von Blutungen im Nasen- und/oder Rachenraum, mit einem beidseitig offenen flexiblen Atemschlauch (2) sowie einem den Atemschlauch (2) mindestens abschnittsweise umhüllenden, mindestens teilweise ausdehnbaren Hüllelement (3, 21), wobei zur Ausdehnung des Hüllelements Medium, insbesondere Luft in einen Raum (14, 29) zwischen dem flexiblen Atemschlauch (2) und dem Hüllelement (21) oder - im Falle eines zweilagig ausgebildeten Hüllelements (3) - zwischen einer äußeren Lage (10) und einer inneren Lage (9) des Hüllelements (3) abwechselnd einbringbar und wieder ausbringbar ist.

2. Vorrichtung nach Anspruch 1, **dadurch gekennzeichnet, dass** der Atemschlauch (2) im Wesentlichen aus Silikon gefertigt ist.

3. Vorrichtung nach einem der Ansprüche 1 oder 2, **dadurch gekennzeichnet, dass** das Hüllelement (3, 21) im Wesentlichen aus Latex gefertigt ist.

4. Vorrichtung nach einem der vorhergehenden Ansprüche, **dadurch gekennzeichnet, dass** das Hüllelement (3, 21) mit dem Atemschlauch (2) mindestens abschnittsweise, insbesondere an seinen Endbereichen fest mit dem Atemschlauch (2) verbunden, insbesondere verklebt oder mittels eines Halterings verbunden ist.

5. Vorrichtung nach einem der vorhergehenden Ansprüche, **dadurch gekennzeichnet, dass** ihr ein Schlauch zum Einbringen von Medium zugeordnet ist, welcher vorzugsweise mit einer Pumpe zum Einpumpen des Mediums verbunden ist.

6. Vorrichtung nach einem der vorhergehenden Ansprüche, **dadurch gekennzeichnet, dass** sie ein Ventil, insbesondere ein Rückschlagventil aufweist.

7. Vorrichtung nach einem der vorhergehenden Ansprüche, **dadurch gekennzeichnet, dass** im Bereich eines distalen, im Einsatzzustand außerhalb des Körpers, insbesondere der Nase eines Patienten befindlichen Endes (5) des Hüllelements (3, 21) ein Abschlussring (6, 22) angeordnet ist, welcher den Atemschlauch (2) umgibt und mit diesem bzw. einer inneren Lage (9) des Hüllelements (3) sowie mit einer äußeren Lage (10) des Hüllelements (3) fest verbunden ist, insbesondere verklebt oder mittels eines Halterings (27, 28) (z. B. Schelle) verbunden ist, wobei der Abschlussring (6) eine Öffnung (8, 26) zum Einbringen bzw. Ausbringen von Medium aufweist.

8. Vorrichtung nach Anspruch 7, **dadurch gekennzeichnet, dass** der Abschlussring (6) doppelwandig ausgebildet ist, wobei die Wände (15, 16) an ihrem einen Ende frei und an ihrem anderen Ende über eine ringförmige Abschlussplatte (7, 25) miteinander verbunden sind, welche Abschlussplatte (7, 25) vorzugsweise eine Aussparung (8, 26) zum Einbringen bzw. Ausbringen von Medium aufweist.
